Europäisches Patentamt

European Patent Office

Office européen des brevets

(19)

(11) Publication number: **0 087 906**
**B1**

(12)

# EUROPEAN PATENT SPECIFICATION

(45) Date of publication of the patent specification:
21.05.86

(51) Int. Cl.⁴: **C 07 C 5/27**, C 07 C 15/08

(21) Application number: 83300906.1

(22) Date of filing: 22.02.83

(54) Xylene isomerization process and catalyst therefor.

(30) Priority: 25.02.82 US 352415

(43) Date of publication of application:
07.09.83 Bulletin 83/36

(45) Publication of the grant of the patent:
21.05.86 Bulletin 86/21

(84) Designated Contracting States:
BE DE FR GB IT NL

(56) References cited:
EP - A - 0 018 498
EP - A - 0 038 141
US - A - 4 098 836
US - A - 4 159 282
US - A - 4 278 565

Chemical Abstracts, vol. 96, no. 1, 4 January 1982,
Columbus, Ohio, USA, S. VISHNOI et al. "Acidic and
catalytic properties of the
calcium-platinum-hydrogen-mordenite system", column
1, abstract no. 12034j
Chemical Abstracts, vol. 82, no. 17, 28 April 1975,
Columbus, Ohio, USA, page 482, column 2, abstract no.
111750g

The file contains technical information submitted after
the application was filed and not included in this
specification

(73) Proprietor: MOBIL OIL CORPORATION, 150 East 42nd
Street, New York New York 10017 (US)

(72) Inventor: Chu, Yung-Feng, 121 Randle Drive, Cherry Hill
New Jersey 08034 (US)
Inventor: Smith, Fritz Arthur, 20 Loder Street, Rye New
York 10580 (US)
Inventor: Chester, Arthur Warren, 517 Country Club
Drive, Cherry Hill New Jersey 08003 (US)

(74) Representative: West, Alan Harry et al, Mobil
Court 3 Clements Inn, London WC2A 2EB (GB)

## Description

This invention relates to a process and catalyst for effecting isomerization of an aromatic $C_8$ mixture comprising ethylbenzene and xylene.

Xylene isomerization in the presence of a catalyst is well known. For example, United States Patent 3,856,872 (Morrison) discloses a method of converting ethylbenzene over an active acid catalyst typified by zeolite ZSM-5. Ethylbenzene disproportionates to benzene and diethylbenzene which are readily separated from xylene by the distillation equipment needed in the loop to remove by-products. It is recognized that the rate of disproportionation of ethylbenzene is related to the rate of conversion of xylene into other compounds, e.g., by disproportionation. United States Patent 3,856,873 (Burress) also describes the reaction of $C_8$ aromatics over ZSM-5 and shows the effect of performing the reaction at various temperatures up to 950°F (510°C) in the absence of metal catalyst and in the absence of hydrogen. In the presence of the catalyst and operating at these higher temperatures, the transalkylation route for xylene losses is reduced; however, xylene losses via the disproportionation route are increased. The xylene loss by-products are mostly trimethyl-benzene, ethyl-xylene, and $C_9+$ aromatics.

United States Patent 4,098,837 (Chu) discloses a zeolite catalyst which incorporates phosphorus and one or more metals, including magnesium, in an effort to elicit a greater para-xylene content from the feed material. United States Patent 4,159,282 (Olson et al) discloses xylene isomerization with a crystalline zeolite catalyst having a crystal size of at least 1 micron. The original alkaline metal of the zeolit may be replaced by ion exchange with suitable ions of Groups 1B to 8. A second metal, e.g., magnesium, may be combined with the zeolite.

United States Patent 4,163,028 (Tabak et al) discloses xylene isomerization and ethylbenzene conversion at temperatures of 800°F (427°C) or higher with ZSM-5 of very high silica/alumina ratio whereby the acid activity is reduced.

United States Patent 4, 278,565 (Chen et al) discloses a zeolite catalyst which has a crystalline size greater than 1 micron, e.g., HZSM-5C and which comprises three metals: a Group IIB metal, a Group VIII metal and magnesium incorporated into the catalyst in the order specified.

EP-A-18498 discloses xylene isomerization over ZSM-5 containing platinum and a further metal, such as barium, but fails to appreciate the importance of a zeolite crystal size.

In accordance with the invention it has now been found to be possible further to reduce xylene by-product formation by incorporating platinum and a Group IIA metal into a zeolite catalyst having a crystal size of 1-20 microns.

Accordingly, the invention resides in one aspect in a process for isomerizing an isomerization feed containing an aromatic $C_8$-mixture of ethylbenzene and xylene which comprises contacting said feed under conversion conditions with a catalyst comprising a crystalline aluminosilicate zeolite having a silica to alumina ratio of at least 12, and a constraint index within the range 1-12 and containing platinum and a Group IIA metal component, characterized in that the crystal size of the zeolite is within the range of 1-20 microns.

The process of the invention utilizes a zeolite catalyst having a crystal size of 1 to 20 microns and preferably 1 to 6 microns. The zeolite catalyst has a silica to alumina ratio of at least 12, a constraint index with in the approximate range of 1 to 12, and contains both platinum and a Group IIA metal, preferably magnesium.

The present process preferably comprises isomerization of an aromatic $C_8$ mixture in the presence of hydrogen such that the hydrogen to hydrocarbon ratio is in the range of 0.1 to 10, more preferably 1 to 5, at a temperature between 500°F and 1000°F (260°C and 538°C), more preferably in excess of 650°F (343°C), at a pressur between 0 and 1500 psig (101 and 10444 kPa), utilizing a feed weight hourly space velocity (WHSV) between 0.5 and 50. The WHSV values are based on the weight of catalyst composition, i.e., total weight of active catalyst and binder thereof.

In the further aspect, the invention resides in a catalyst comprising a crystalline aluminosilicate zeolite having a crystal size within the range of 1-20 microns, a silica to alumina ratio of at least 12 and a constraint index within the range 1 to 12, said zeolite containing platinum and magnesium.

It is contemplated that any aromatic $C_8$ mixture containing ethylbenzene and xylene in which the para-xylene content is less than equilibrium may be used as the feed for the present process. Generally, the mixture will have an ethylbenzene content in the range of 5-50 wt. %, an ortho-xylene content in the range of 0-15 wt. %, and a meta-xylene content in the range of 0-70 wt. %. The feed may also contain non-aromatic hydrocarbons, i.e., naphthenes and paraffins. In preferred embodiment, a mixture of $C_8$ aromatics is processed so as to reduce the ethylbenzene content of the mixture and increase its content of para-xylene.

The feed is brought into contact, under conversion conditions, with a catalyst comprising a crystalline aluminosilicate zeolite having a a silica to alumina ratio of at least 12 and a constraint index within the range of 1 to 12. Such zeolites are well known in the art and suitable descriptions may be found in United States Patents 4,159,282; 4,163,028; and 4,278,565. The preferred crystalline aluminosilicate zeolites are ZSM-5, ZSM-11, ZSM-12, ZSM-35 and ZSM 38, with ZSM-5 being particularly preferred.

The crystal size of the crystalline aluminosilicate zeolite employed herein is in the range of 1 to 20 microns and most preferably in the range of 1 to 6 microns. The longer diffusional paths of zeolite crystals in excess of 1 micron reduce by-product formation thus decreasing xylene loss compared to zeolite crystals in the 0.5 micron range. Further, with the use of crystals within such a size range distinctly higher selectivity for production of para-xylene has been observed as compared with comparable use of smaller size crystals.

The preferred catalyst contains from 0.5% to 10%, more preferably 1% to 5%, by weight of the Group IIA component, most preferably magnesium, and 0.01% to 1%, more preferably 0.05% to 1%, by weight of platinum. The catalyst is prepared by conventional techniques including impregnation, base exchange, drying and air calcination. If desired the catalyst can be steamed for 1 or more hours at temperatures upwards of 300°F (149°C), the time, pressure and temperature being inter-related such that less time is required at higher temperatures and/or pressures.

The feed is contacted with the above-described catalyst at a temperature between 500°F and 950°F (260°C and 510°C); a contact time equivalent to or the same as a weight hourly space velocity (WHSV) of 0.5 to 50, preferably 5 to 25, and a pressure of 0 to 1500 psig (101 to 10444 kPa), preferably between 20 and 400 psig (239 and 2859 kPa).

The reaction product effluent contains ethane, benzene, toluene and other aromatic hydrocarbons with high selectivity for para-xylene. The combination of the larger-size ZSM-5 crystal with two metals, e.g., platinum and magnesium, has been found to reduce the formation of by-products, such as trimethyl-benzene, ethyl-xylene, $C_9+$ aromatics, and increase benzene formation.

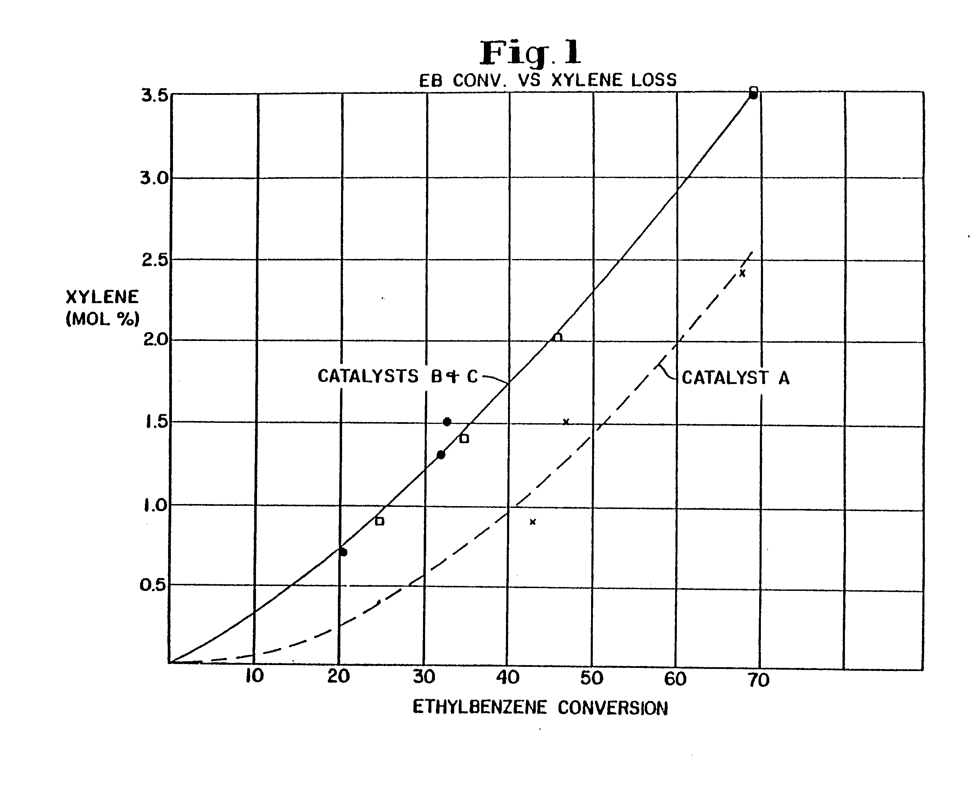

The following example serves to illustrate the process of this invention with reference to the accompanying drawing which compares graphically the amounts of ethylbenzene conversion and xylene loss for three isomerization processes.

## EXAMPLE
### Catalyst Preparation

HZSM-5 having an average crystal size of 2 microns was conventionally produced using tetrapropylammonium and sodium cations or tetra-methylammonium and sodium cations. Examples of the production of such zeolites are given in British Patent No. 2,002,733.

The resultant HZSM-5 crystals were ion exchanged with ammonium nitrate solution, dried and then introduced into a reactor through which a $Mg(NO_3)_2$ solution was circulated until about 2.4% Mg was incorporated onto the catalyst. The catalyst was then dried at 250°F (121°C) and subsequently calcined by heating to 850°F (455°C) in $N_2$ followed by heating to 1000°F (538°C) for 2 hours. The resultant Mg ZSM-5 was then agitated with an aqueous solution of $[Pt(NH_3)_4]Cl$ containing the equivalent of 0.1 wt% Pt, dried at 250°F (121°C) and then air calcined at 1000°F (538°C) for 3 hours. This catalyst will hereinafter be referred to as Catalyst A.

### Isomerization

A feed comprising a blend of ethylbenzene and mixed xylenes and having the composition set forth in Table I below, was tested in three different pressurized xylene isomerization units. In Run 1, the feed was passed over a catalyst comprising a platinum impregnated HZSM-5 (no magnesium) having an average crystal size of 0.1 microns, hereinafter referred to as Catalyst 8, at a temperature of 853°F (456°C), a pressure of 100 psig (791 kPa) and WHSV of 14.7. In Run 2, the feed was passed over a catalyst comprising a platinum-impregnated HZSM-5 (no magnesium) having an average crystal size of 0.1 microns, hereinafter referred to as Catalyst C, at a temperature of 677°F (358°C), a pressure of 100 psig (791 kPa) and a WHSV of 7.0. In Run 3, the feed was passed over Catalyst A at a temperature of 700°F (371°C), a pressure of 100 psig (791 kPa) and a WHSV of 6.5. The product compositions and selectivities obtained in each instance are given in Table I, in which:

-Xylene Loss (mole %) is calculated by:

$$\text{Xylene loss} = \frac{[T]_{P-F} + [TMB]_{P-F} + [EX]_{P-F}}{[P-X + M-X + O-X]_F} \times 100$$

where T = Toluene, TMB = trimethylbenzene, EX = ethylxylene, P-X, M-X and O-X = para-, meta-, and ortho-xylene, respectively,

P = product, F = feed, [ ] = mole %

$H_2$/HC Molar Ratio is the molar ratio of $H_2$ and hydrocarbons in the feed.

EB Conv. Wt. % is the weight percent conversion of ethylbenzene.

P-Xylene Equil. Approach % is the weight percent equilibrium approach of P-xylene.

$C_2=/C_2$ is the molar ratio of ethylene and ethane in the product.

$\Delta$ EB is the amount of ethylbenzene converted.

BZ is the amount (moles) of benzene formed in the product.

$C_2/\Delta$ EB is the molar ratio of ethane produced per mole of ethylbenzene converted.

BZ/$\Delta$ EB is the molar ratio of benzene produced per mole of ethylbenzene converted.

$C_1$-$C_4$ and $C_5$-$C_9$ N.A. designates the $C_1$-$C_4$ and $C_5$-$C_9$ non-aromatics respectively formed in the product.

**TABLE I**

| | | Run No. | | | | |
|---|---|---|---|---|---|---|
| | | 1 | | 2 | | 3 |
| Catalyst | | B | | C | | A |
| Temp., °F (°C) | | 853 (456) | | 677 (358) | | 700 (371) |
| Pressure, psig (kPa) | | 100 (791) | | 100 (791) | | 100 (791) |
| WHSV | | 14.7 | | 7.0 | | 6.5 |
| $H_2$/HC Molar Ratio | | 2.2 | | 2.0 | | 2.0 |
| Product Analysis | | | | | | |
| (wt. %) | feed | | feed | | feed | |
| $C_1$-$C_4$ N.A. | | 0.7 | | 0.7 | | 1.4 |
| $C_5$-$C_9$ N.A. | | 0.0 | | 0.1 | | 0.7 |
| BZ | | 2.2 | | 1.6 | | 2.5 |
| Toluene | 1.5 | 2.1 | 1.5 | 2.1 | 1.5 | 1.9 |
| Ethylbenzene | 8.1 | 5.2 | 8.6 | 5.7 | 9.0 | 5.1 |
| P-Xylene | 9.2 | 21.3 | 9.4 | 21.5 | 8.8 | 21.4 |
| M-Xylene | 61.3 | 46.8 | 60.8 | 47.1 | 61.4 | 47.8 |
| O-Xylene | 19.9 | 20.7 | 19.7 | 19.9 | 19.3 | 18.6 |
| Ethyl-toluene | | 0.1 | | 0.3 | | 0.2 |
| Tri-methylbenzene | | 0.7 | | 0.5 | | 0.3 |
| Di-ethylbenzene | | > 0 | | 0.2 | | 0.2 |
| Ethyl-xylene | | 0.1 | | 0.3 | | > 0 |
| $C_{10}$ | | - | | - | | - |
| EB Conv. Wt.% | | 35 | | 33 | | 43 |
| Xylene Loss, Mole % | | 1.4 | | 1.5 | | 0.9 |
| P-Xylene Equil. Approach % | | 105 | | 105 | | 105 |
| $C_2$=/$C_2$ | | 0 | | 0 | | 0 |
| $C_2$/$\Delta$ EB | | 0.8 | | 0.5 | | 0.9 |
| BZ/$\Delta$ EB | | 1.1 | | 0.8 | | 0.9 |

0 087 906

A comparison of the xylene selectivities of the three isomerization processes utilized in the above Example is shown in the drawing. Referring more particularly to this Figure, selectivity is expressed in terms of the ratio of xylene loss to relative ethylbenzene conversion. It will be evident that there is a marked improvement in selectivity obtained utilizing Catalyst A over that obtained with conventional catalysts exemplified by Catalysts B and C. At about 50% ethylbenzene conversion, the xylene loss advantage for the improved Catalyst A is about 0.9% per pass.

**Claims:**

1. A process for isomerizing an isomerization feed containing an aromatic $C_8$-mixture of ethylbenzene and xylene which comprises contacting said feed under conversion conditions with a catalyst comprising a crystalline aluminosilicate zeolite having a silica to alumina ratio of at least 12, and a constraint index within the range 1-12 and containing platinum and a Group IIA metal component, characterized in that the crystal size of the zeolite is within the range of 1-20 microns.

2. The process of claim 1 wherein said conversion conditions include a temperature of from 500°F to 1000°F (260°C to 538°C), a pressure of 0 to 1500 psig (101 to 10444 kPa) and a weight hourly space velocity of between 0.5 and 100.

3. The process of claim 1 or claim 2 wherein the Group IIA metal is magnesium.

4. The process of any preceding claim wherein the zeolite is ZSM-5, ZSM-11, ZSM-12, ZSM-35, or ZSM-38.

5. The process of any preceding claim wherein said crystalline aluminosilicate zeolite is ZSM-5.

6. The process of any preceding claim wherein the crystalsize of said crystalline aluminosilicate zeolite is within the range of 1 to 6 microns.

7. A catalyst for use in the process as claimed in any preceding claim comprising a crystalline aluminosilicate zeolite having a crystal size within the range of 1 to 20 microns, a silica to alumina ratio of at least 12 and a constraint index within the range of 1 to 12, said zeolite containing platinum and magnesium.

8. A catalyst as claimed in claim 7 wherein the platinum constitutes between 0.1% and 1% by weight of the catalyst and the magnesium constitutes between 0.5% and 10% by weight of the catalyst.

**Patentansprüche**

1. Verfahren zur Isomerisierung einer Isomerisierungszufuhr, die eine aromatische $C_8$-Mischung von Ethylbenzol und Xylol enthält, wobei das Verfahren das Inkontaktbringen der Zufuhr unter Umwandlungsbedingungen mit einem Katalysator umfaßt, der einen kristallinen Aluminosilikatzeolith mit einem Siliciumdioxid/Aluminiumoxid-Verhältnis von mindestens 12 und einem Zwangsindex im Bereich von 1 bis 12 umfaßt, der Platin und eine Metallkomponente der Gruppe IIA enthält, dadurch <u>gekennzeichnet</u>, daß die Kristallgröße des Zeoliths im Bereich von 1 bis 20 μm liegt.

2. Verfahren nach Anspruch 1, worin die Umwandlungsbedingungen eine Temperatur von 500 bis 1000°F (260 bis 538°C), einen Druck von 0 bis 1500 psig (101 bis 10 444 kPa) und eine stündliche Raumgewichtsgeschwindigkeit von zwischen 0,5 und 100 einschließen.

3. Verfahren nach Anspruch 1 oder 2, worin das Metall der Gruppe IIA Magnesium ist.

4. Verfahren nach einem der vorstehenden Ansprüche, worin der Zeolith ZSM-5, ZSM-11, ZSM-12, ZSM-35 oder ZSM-38 ist.

5. Verfahren nach einem der vorstehenden Ansprüche, worin der kristalline Aluminosilikatzeolith ZSM-5 ist.

6. Verfahren nach einem der vorstehenden Ansprüche, worin die Kristallgröße des kristallinen Aluminosilikatzeoliths im Bereich von 1 bis 6 μm liegt.

7. Katalysator zur Verwendung in einem Verfahren nach einem der vorstehenden Ansprüche, der einen kristallinen Aluminosilikatzeolith mit einer Kristallgröße im Bereich von 1 bis 20 μm, einem Siliciumdioxid/Aluminiumoxid-Verhältnis von mindestens 12 und einem Zwangsindex im Bereich von 1 bis 12 umfaßt, wobei der Zeolith Platin und Magnesium enthält.

8. Katalysator nach Anspruch 7, worin Platin zwischen 0,1 und 1 Gew.-% des Katalysators bildet und Magnesium zwischen 0,5 und 10 Gew.-% des Katalysators bildet.

**Revendications**

1. Procédé pour l'isomérisation d'une charge d'isomérisation contenant un mélange aromatique en $C_8$ d'éthylbenzène et de xylène qui consiste à mettre en contact ladite charge dans les conditions de conversion avec un catalyseur comprenant une zéolite d'aluminosilicate cristallin ayant un rapport silice/ alumine d'au moins 12 et un indice de contrainte dans la gamme de 1-12 et contenant du platine et un composant métal du groupe IIA, caractérisé en ce que la dimension de cristaux de la zéolite est dans la gamme de 1-20 μm.

5

2. Procédé selon la revendication 1, dans lequel lesdites conditions de conversion comprennent une température de 260 à 538°C (500 à 1000°F), une pression de 101 à 10444 kPa (0 à 1500 psig) et une vitesse spatiale horaire en poids entre 0,5 et 100.

3. Procédé selon la revendication 1 ou 2, dans lequel le métal du groupe IIA est le magnésium.

4. Procédé selon l'une quelconque des revendications precédentes, dans lequel la zéolite est la ZSM-5, la ZSM-11, la ZSM-12, la ZSM-35 ou la ZSM-38.

5. Procédé selon l'une quelconque des revendications précédentes, dans lequel ladite zéolite d'aluminosilicate cristallin est la ZSM-5.

6. Procédé selon l'une quelconque des revendications précédentes, dans lequel la dimension de cristaux de ladite zéolite d'aluminosilicate cristallin est dans la gamme de 1 à 6 μm.

7. Catalyseur pour l'utilisation dans le procédé selon l'une quelconque des revendications précédentes, comprenant une zéolite d'aluminosilicate cristallin ayant une dimension de cristaux dans la gamme de 1 à 20 μm, un rapport silice/alumine d'au moins 12 et un indice de contrainte dans la gamme de 1 à 12, ladite zéolite contenant du platine et du magnésium.

8. Catalyseur selon la revendication 7, dans le platine constitue entre 0,1 et 1 % en poids du catalyseur et le magnésium entre 0,5 et 10 % en poids du catalyseur.

# Fig. 1

## EB CONV. VS XYLENE LOSS

XYLENE (MOL %)

CATALYSTS B + C

CATALYST A

ETHYLBENZENE CONVERSION